# EUROPEAN PATENT SPECIFICATION

(11) **EP 3 829 523 B1**
(45) Date of publication and mention of the grant of the patent: **21.08.2024**
(21) Application number: 18924134.2
(22) Date of filing: 28.06.2018
(51) Int. Cl.: A61K 8/02, A61K 8/04, A61K 8/37, A61K 8/88, A61K 8/92, A61Q 1/00, A61Q 5/00, A61Q 19/00

(54) **OIL-IN-WATER COMPOSITION COMPRISING A HYDROPHOBIC POLYMER**
ÖL-IN-WASSER-ZUSAMMENSETZUNG MIT EINEM HYDROPHOBEN POLYMER
COMPOSITION D'HUILE DANS L'EAU COMPRENANT UN POLYMÈRE HYDROPHOBE

(43) Date of publication of application: 09.06.2021
(73) Proprietor: L'OREAL, 75008 Paris (FR); Wang, Brave, Shanghai 201206 (CN)
(72) Inventor: WANG, Brave, Shanghai 201206 (CN); CHEN Daniel, 201206 Shanghai (CN); CHEN, Francis, 201206 Shanghai (CN)
(74) Representative: Lavoix
(86) International application number: PCT/CN2018/093226
(87) International publication number: WO 2020/000283

(56) References cited:
- WO-A2-2010/006854
- US-A1- 2002 187 170
- US-A1- 2013 158 130
- US-A1- 2015 050 366
- US-A1- 2016 030 304
- US-A1- 2016 367 454

## Description

The present invention is defined in the appended claims and concerns a composition in the form of an oil-in-water composition, comprising at least one hydrophobic polymer. The present invention also concerns a cosmetic composition useful for personal skin care.

The development of formulations dedicated to caring for and/or making up the skin and/or lips, is permanent. Said formulations have to show satisfactory properties in terms of sensory feeling after application.

For the development of such formulations, visual aspects are also of importance for the consumers. In particular, as visual aspects, the formation of water droplets when applying the composition onto the skin is highly desired by the consumers. To this date, products having this visual effect are water-in-oil emulsions which have the drawbacks of giving an oily feeling on the skin.

There is thus a need for a composition for skin care having satisfying visual effects as well as satisfying sensory feelings and properties.

The aim of the present invention is thus to provide a composition with a satisfying sensory feeling, without any oily feeling, and having good hydration properties, as well as satisfying stability properties.

The aim of the present invention is also to provide a composition, useful in particular as a gel, cream, lotion or serum, having a thick texture, being easy to quickly break upon application, and providing the consumers with a transformative experience with visible water droplets on top of an oily film on the skin.

Another aim of the present invention is to provide a composition with very satisfying hydration properties, which quickly breaks into separated aqueous and lipophilic phases upon application, and thus delivering both hydrophilic and lipophilic beneficial ingredients with a watery and fresh sensation.

Therefore, the present invention relates to a composition, preferably a cosmetic composition, in the form of an oil-in-water emulsion, comprising:
- a dispersed fatty phase comprising:
   . at least one triglyceride made of glycerol and fatty acids, preferably at least one triglyceride made of glycerol and fatty acids with saturated or unsaturated, linear or branched, chains comprising from 8 to 36 carbon atoms, and
   . at least one hydrophobic polymer selected from the ester-terminated poly(ester-amide)s;
      wherein said dispersed fatty phase comprises at least 10% by weight of triglyceride(s) in relation to the total weight of said dispersed fatty phase; and
- a continuous aqueous phase comprising at least one hydrophilic gelling agent selected from the group consisting of the polymers comprising at least one monomer being acrylamido-2-methylpropanesulfonic acid, the optionally modified carboxyvinyl polymers, and mixtures thereof.

The composition according to the invention comprises a dispersed fatty phase (or lipophilic or hydrophobic phase) and a continuous aqueous phase (or hydrophilic phase) and it is in the form of an oil-in-water emulsion.

The composition of the invention is stable over time. Within the present application, a stable composition is a composition wherein there is no oil/water leakage, that is to say wherein the composition remains in the form of an emulsion.

According to an embodiment, the composition of the invention is stable at 25°C for at least one week. According to an embodiment, the composition of the invention is stable at 4°C for at least one week. According to an embodiment, the composition of the invention is stable at 45°C for at least one week.

The present invention is based on the fact that the ester-terminated polyesteramide texturizes the oil phase into a thixotropic viscos-elastic texture which has much less chance to coalesce than liquid oil when dispersed in aqueous phase, making the product stable when stored. In the condition of application (rubbing, electrolyte change, etc.), the absence of conventional surfactant and thixotropy of the gels make it easy to break into continuous oily phase and aqueous phase, providing the consumers a transformative experience with visible water drops on top of an oily film on the skin. The sensation of application is watery and fresh, while the hydrophobic beneficial ingredients are delivered.

The composition of the invention is advantageous in that it quickly breaks into separated aqueous and lipophilic phases upon application, delivering both hydrophilic and lipophilic beneficial ingredients with a watery and fresh sensation.

Moreover, the composition of the invention has a thick texture but it breaks quickly upon application and rubbing with visible spherical water droplets.

In that which follows and unless otherwise indicated, the limits of a range of values are included within this range, in particular in the expressions "of between" and "ranging from ... to ...".

Moreover, the expression "at least one" used in the present description is equivalent to the expression "one or more".

Throughout the instant application, the term "comprising" is to be interpreted as encompassing all specifically mentioned features as well optional, additional, unspecified ones. As used herein, the use of the term "comprising" also discloses the embodiment wherein no features other than the specifically mentioned features are present (i.e. "consisting of").

### Fatty phase

The composition of the invention comprises a dispersed fatty phase.

According to an embodiment, the amount of dispersed fatty phase is less than or equal to 30% by weight relative to the total weight of the composition.

### Triglyceride(s)

The fatty phase of the composition of the invention comprises at least one triglyceride or a mixture of several triglycerides.

As mentioned above, according to the invention, the triglycerides are made of glycerol and fatty acids, wherein said fatty acids have a saturated or unsaturated, linear or branched, chain comprising from 8 to 36 carbon atoms.

Preferably, the composition of the invention comprises a mixture of triglycerides as defined above.

According to an embodiment, the triglycerides are chosen from triglycerides constituted of glycerol and fatty acid esters, in particular in which the fatty acids may have chain lengths ranging from C₈ to C₃₆, and in particular from C₁₈ to C₃₆.

Preferably, the composition of the invention comprises caprylic/capric acid triglycerides or C₁₈-C₃₆ acid triglycerides.

The composition of the invention may comprise oils which may be heptanoic or octanoic triglycerides, or alternatively caprylic/capric acid triglycerides, such as those sold by STEARINERIE DUBOIS or those sold under the names MIGLYOL 810^{®}, 812^{®} and 818^{®} by DYNAMIT NOBEL.

As mentioned above, in the composition of the invention, the amount of triglyceride(s) is of at least 10% by weight in relation to the total weight of the fatty phase of said composition.

According to an embodiment, the amount of triglyceride(s) is comprised from 10% to 90%, preferably from 30% to 70%, and more preferably from 40% to 60%, by weight in relation to the total weight of said dispersed fatty phase.

According to an embodiment, the amount of triglyceride(s) is comprised from 1% to 20%, preferably from 2% to 15%, and more preferably from 5% to 10%, by weight in relation to the total weight of said composition.

### Hydrophobic polymer

The composition of the invention comprises at least one hydrophobic polymer selected from the ester-terminated poly(ester-amide)s.

By "hydrophobic" it may be meant a polymer which is repelled from water, i.e. which is not miscible with water.

By "ester-terminated" polymer it may be meant a polymer comprising at least one chemical function ester (Rₐ-C(O)-O-R'ₐ) as a terminal moiety. For example, such polymers and their methods of preparation are described in the international application WO 02/092663.

In one embodiment, said polymer comprises at least one dimer dilinoleate unit, optionally hydrogenated. Preferably, the hydrophobic polymer is bis-stearyl ethylenediamine/neopentyl glycol/hydrogenated dimer dilinoleate copolymer. Particular mention may be made of bis-stearyl ethylenediamine/neopentyl glycol/hydrogenated dimer dilinoleate copolymer, also called polyamide-8, commercialized by Arizona chemical under the name Sylvaclear^{™} C75V or by Croda, Inc., under the name OleoCraft LP-20 (CAS RN is 678991-29-2).

In one embodiment, the amount of the hydrophobic polymer is comprised from 0.1% to 10% by weight, more preferably from 0.3% to 5% by weight, based on the total weight of the composition.

According to an embodiment, the amount of said hydrophobic polymer is comprised from 0.3% to 10% by weight, more preferably from 0.5% to 5% by weight, and most preferably from 1% to 3% by weight, based on the total weight of the composition.

### Additional hydrophobic ingredients

The dispersed fatty phase of the invention may further comprise at least one hydrophobic additional ingredient.

According to an embodiment, the hydrophobic additional ingredients are chosen from hydrocarbonated compounds comprising Cs-Cse saturated or unsaturated carbon chains. Preferably, said compounds are chosen from linear or branched hydrocarbons of mineral or synthetic origin.

Preferably, the fatty phase of the composition of the invention comprises at least one linear or branched hydrocarbon of mineral or synthetic origin, such as liquid paraffins and derivatives thereof, petroleum jelly, polydecenes, polybutenes, hydrogenated polyisobutene such as Parleam, or squalane. Preferably, the fatty phase comprises squalane and/or hydrogenated polyisobutene.

Other examples of hydrocarbonated compounds comprising C₈-C₃₆ saturated or unsaturated carbon chains are waxes, in particular synthetic waxes, and mineral oils such as liquid paraffins and derivatives thereof.

Preferably, the waxes can be hydrocarbon, fluorinated and/or silicone waxes and be of plant, mineral, animal, and/or synthetic origin.

Preferably, the wax or waxes comprise one or several polar and/or non-polar waxes chosen from among carnauba wax, candellila wax, natural (or whitened) beeswax and synthetic beeswax, paraffin and hydrocarbon wax, and mixtures thereof.

According to a preferred embodiment, the wax is a polymethylene wax such as Cirebelle 303 sold by Cirebelle.

According to an embodiment, the hydrophobic additional ingredients are chosen from the plant oils.

As plant oils, one may mention the followings: shea oil, alfalfa oil, poppy seed oil, pumpkin oil, millet oil, barley oil, quinoa oil, rye oil, candlenut oil, passionflower oil, shea butter, aloe oil, sweet almond oil, peach kernel oil, groundnut oil, argan oil, avocado oil, baobab oil, barrage oil, broccoli oil, calendula oil, camelina oil, canola oil, carrot oil, safflower oil, hemp oil, rapeseed oil, cotton seed oil, coconut oil, marrow seed oil, wheat germ oil, jojoba oil, lily oil, macadamia oil, corn oil, meadowfoam oil, St. John's Wort oil, monoi oil, hazelnut oil, apricot kernel oil, nut oil, olive oil, evening primrose oil, palm oil, blackcurrant seed oil, kiwi seed oil, grape seed oil, pistachio oil, pumpkin oil, winter squash oil, quinoa oil, musk rose oil, sesame oil, soya oil, sunflower oil, castor oil and watermelon oil, and mixtures thereof.

According to an embodiment, the composition of the invention comprises at least one hydrocarbonated compound comprising Cs-Cse saturated or unsaturated carbon chains as defined above and/or at least one plant oil as defined above.

According to an embodiment, the dispersed fatty phase comprises at least one hydrocarbonated compound comprising C₈-C₃₆ saturated or unsaturated carbon chains as defined above in an amount comprised between 10% and 90%, preferably from 30% to 70%, and more preferably from 40% to 60%, by weight in relation to the total weight of said dispersed fatty phase.

According to an embodiment, the dispersed fatty phase comprises at least one plant oil as defined above, preferably shea butter, in an amount comprised between 10% and 90%, preferably from 30% to 70%, and more preferably from 40% to 60%, by weight in relation to the total weight of said dispersed fatty phase.

Preferably, the hydrophobic additional ingredient is chosen from the linear or branched hydrocarbons of mineral or synthetic origin such as squalane, the plant oils such as shea butter, and mixtures thereof.

According to an embodiment, the amount of linear or branched hydrocarbons of mineral or synthetic origin such as squalane is comprised from 1% to 20%, preferably from 2% to 15%, and more preferably from 5% to 10%, by weight in relation to the total weight of said composition.

According to an embodiment, the amount of plant oil such as shea butter is comprised from 1% to 20%, preferably from 2% to 15%, and more preferably from 5% to 10%, by weight in relation to the total weight of said composition.

### Aqueous phase

The composition of the invention comprises a continuous aqueous phase.

According to an embodiment, the amount of continuous aqueous phase is greater than or equal to 50% by weight relative to the total weight of the composition.

Said aqueous phase is preferably present in an amount ranging from 50% to 99% by weight, more preferably from 50% to 85% by weight of the total weight of the composition.

The continuous aqueous phase may comprise water, as well as preferably at least one organic solvent miscible with water or mixtures thereof.

Preferably, the continuous aqueous phase comprises at least one water-miscible organic solvent (at room temperature - 25°C) such as for example monoalcohols having from 2 to 6 carbon atoms such as ethanol, isopropanol; polyols notably having from 2 to 20 carbon atoms, preferably from 2 to 10 carbon atoms, and preferentially having from 2 to 6 carbon atoms, such as glycerol, propylene glycol, butylene glycol, pentylene glycol, hexylene glycol, caprylylglycol, dipropylene glycol, diethylene glycol; glycol ethers (notably having from 3 to 16 carbon atoms) such as mono-, di- or tri-propylene glycol (C₁-C₄)alkyl ethers, mono-, di- or triethylene glycol (C₁-C₄) alkyl ethers; and mixtures thereof.

According to one embodiment, the aqueous phase of the compositions according to the invention comprises glycerol, phenoxyethanol, or a mixture thereof.

The continuous aqueous phase of the composition of the invention preferably comprises water and at least a polyol, preferably glycerol or propylene glycol, and/or a monoalcohol, preferably ethanol.

Preferably, the continuous aqueous phase comprises water, a mixture of polyols, preferably glycerol and propylene glycol.

Preferably, water is present in an amount ranging from 20% to 80% by weight, more preferably from 30% to 70% by weight of the total weight of the composition.

Preferably, the organic solvent(s) miscible with water is(are) present in an amount ranging from 5% to 80% by weight, more preferably from 7% to 20% by weight of the total weight of the composition.

### Hydrophilic gelling agent(s)

According to an embodiment, the composition of the present invention comprises, in a continuous aqueous phase, at least one hydrophilic gelling agent.

For the purposes of the present invention, the term "hydrophilic gelling agent" means a compound that is capable of gelling the aqueous phase of the compositions according to the invention.

The gelling agent is hydrophilic and is thus present in the aqueous phase of the composition.

The gelling agent may be water-soluble or water-dispersible.

According to the invention, the hydrophilic gelling agent may be a polymer comprising at least one monomer being acrylamido-2-methylpropanesulfonic acid (AMPS^{®}) (or AMPS^{®} polymers), an optionally modified carboxyvinyl polymer, or a mixture thereof.

### AMPS^{®} polymers

According to an embodiment, the aqueous phase comprises at least one polymer comprising at least one monomer being acrylamido-2-methylpropanesulfonic acid (AMPS^{®}).

In particular, the AMPS^{®} polymers in accordance with the invention are crosslinked or non-crosslinked homopolymers or copolymers comprising at least the acrylamido-2-methylpropanesulfonic acid (AMPS^{®}) monomer, in a form partially or totally neutralized with a mineral base other than ammonia, such as sodium hydroxide or potassium hydroxide.

They are preferably totally neutralized or virtually totally neutralized, i.e. at least 90% neutralized.

These AMPS^{®} polymers according to the invention may be crosslinked or non-crosslinked.

When the polymers are crosslinked, the crosslinking agents may be chosen from the polyolefinically unsaturated compounds commonly used for the crosslinking of polymers obtained by free-radical polymerization. Examples of crosslinking agents that may be mentioned include divinylbenzene, diallyl ether, dipropylene glycol diallyl ether, polyglycol diallyl ethers, triethylene glycol divinyl ether, hydroquinone diallyl ether, ethylene glycol or tetraethylene glycol di(meth)acrylate, trimethylolpropane triacrylate, methylenebisacrylamide, methylenebismethacrylamide, triallylamine, triallyl cyanurate, diallyl maleate, tetraallylethylenediamine, tetraallyloxyethane, trimethylolpropane diallyl ether, allyl (meth)acrylate, allylic ethers of alcohols of the sugar series, or other allylic or vinyl ethers of polyfunctional alcohols, and also allylic esters of phosphoric and/or vinylphosphonic acid derivatives, or mixtures of these compounds.

According to one embodiment of the invention, the crosslinking agent is chosen from methylenebis-acrylamide, allyl methacrylate and trimethylolpropane triacrylate (TMPTA). The degree of crosslinking generally ranges from 0.01 mol% to 10 mol% and more particularly from 0.2 mol% to 2 mol% relative to the polymer.

The AMPS^{®} polymers in accordance with the invention are water-soluble or water-dispersible. In this case they are:
- either "homopolymers" comprising only AMPS^{®} monomers and, if they are crosslinked, one or more crosslinking agents such as those defined above;
- or copolymers obtained from AMPS^{®} and from one or more hydrophilic or hydrophobic ethylenically unsaturated monomers and, if they are crosslinked, one or more crosslinking agents such as those defined above. When the said copolymers comprise hydrophobic ethylenically unsaturated monomers, these monomers do not comprise a fatty chain and are preferably present in small amounts.

For the purposes of the present invention, the term "fatty chain" means any hydrocarbon-based chain containing at least 7 carbon atoms.

The term "water-soluble or water-dispersible" means polymers which, when introduced into an aqueous phase at 25°C, to a mass concentration equal to 1%, make it possible to obtain a macroscopically homogeneous and transparent solution, i.e. a solution that has a maximum light transmittance value, at a wavelength equal to 500 nm, through a sample 1 cm thick, of at least 60% and preferably of at least 70%.

The "homopolymers" according to the invention are preferably crosslinked and neutralized, and they may be obtained according to the preparation process comprising the following steps:
(a) the monomer such as AMPS^{®} in free form is dispersed or dissolved in a solution of tert-butanol or of water and tert-butanol;
(b) the solution or dispersion of monomer obtained in (a) is neutralized with one or more mineral or organic bases, preferably ammonia NH₃, in an amount making it possible to obtain a degree of neutralization of the sulfonic acid functions of the polymer ranging from 90% to 100%;
(c) the crosslinking monomer(s) is(are) added to the solution or dispersion obtained in (b);
(d) a standard free-radical polymerization is performed in the presence of free-radical initiators at a temperature ranging from 10 to 150°C; the polymer precipitates in the solution or dispersion based on tert-butanol.

The AMPS^{®} homopolymers according to the invention are preferably optionally crosslinked and/or neutralized 2-acrylamido-2-methylpropanesulfonic acid homopolymers, for instance the poly(2-acrylamido-2-methylpropanesulfonic acid) sold by the company Clariant under the name Hostacerin AMPS^{®} (CTFA name: ammonium polyacryldimethyltauramide).

The water-soluble or water-dispersible AMPS^{®} copolymers according to the invention contain preferably water-soluble ethylenically unsaturated monomers, hydrophobic monomers or mixtures thereof.

The water-soluble comonomers may be ionic or nonionic.

Among the ionic water-soluble comonomers, examples that may be mentioned include the following compounds and the salts thereof:
- (meth)acrylic acid,
- styrenesulfonic acid,
- vinylsulfonic acid and (meth)allylsulfonic acid,
- vinylphosphonic acid,
- maleic acid,
- itaconic acid,
- crotonic acid,
- the water-soluble vinyl monomers of formula (A) below: 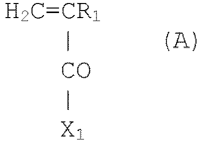
in which:
- R₁ is chosen from H, -CH₃, -C₂H₅ and -C₃H₇ and
- X₁ is chosen from alkyl ethers of -OR₂ type in which R₂ is a linear or branched, saturated or unsaturated hydrocarbon-based radical containing from 1 to 6 carbon atoms, substituted with at least one sulfonic (-SO₃-) and/or sulfate (-SO₄-) and/or phosphate (-PO₄H₂-) group.

Among the nonionic water-soluble comonomers, examples that may be mentioned include:
- (meth)acrylamide,
- N-vinylacetamide and N-methyl-N-vinylacetamide,
- N-vinylformamide and N-methyl-N-vinylformamide,
- maleic anhydride,
- vinylamine,
- N-vinyllactams comprising a cyclic alkyl group containing 4 to 9 carbon atoms, such as n-vinylpyrrolidone, N-butyrolactam and N-vinylcaprolactam,
- vinyl alcohol of formula CH₂=CHOH,
- the water-soluble vinyl monomers of formula (B) below: 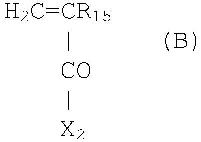
in which:
- R₁₅ is chosen from H, -CH₃, -C₂H₅ and -C₃H₇
- X₂ is chosen from - alkyl ethers of -OR₁₆ type in which R₁₆ is a linear or branched, saturated or unsaturated hydrocarbon-based radical containing from 1 to 6 carbons, optionally substituted with a halogen atom (iodine, bromine, chlorine or fluorine); a hydroxyl group (-OH); ether.

Mention is made, for example, of glycidyl (meth)acrylate, hydroxyethyl methacrylate and (meth)acrylates of ethylene glycol, of diethylene glycol or of polyalkylene glycol.

Among the fatty-chain-free hydrophobic comonomers, examples that may be mentioned include:
- styrene and its derivatives, such as 4-butylstyrene, α-methylstyrene and vinyltoluene,
- vinyl acetate of formula CH₂=CH-OCOCH₃;
- vinyl ethers of formula CH₂=CHOR in which R is a linear or branched, saturated or unsaturated hydrocarbon-based radical containing from 1 to 6 carbons;
- acrylonitrile,
- caprolactone,
- vinyl chloride and vinylidene chloride,
- silicone derivatives, which lead to silicone polymers after polymerization, such as methacryloxypropyltris(trimethylsiloxy)silane and silicone methacrylamides,
- the hydrophobic vinyl monomers of formula (C) below: 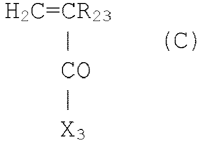
in which:
- R₂₃ is chosen from H, -CH₃, -C₂H₅ and -C₃H₇
- X₃ is chosen from alkyl ethers of -OR₂₄ type in which R₂₄ is a linear or branched, saturated or unsaturated hydrocarbon-based radical containing from 1 to 6 carbon atoms.

Mention is made, for example, of methyl methacrylate, ethyl methacrylate, n-butyl (meth)acrylate, tert-butyl (meth)acrylate, cyclohexyl acrylate and isobornyl acrylate and 2-ethylhexyl acrylate.

The water-soluble or water-dispersible AMPS^{®} polymers of the invention preferably have a molar mass ranging from 50 000 g/mol to 10 000 000 g/mol, preferably from 80 000 g/mol to 8 000 000 g/mol and even more preferably from 100 000 g/mol to 7 000 000 g/mol.

Examples of water-soluble or water-dispersible AMPS^{®} homopolymers in accordance with the invention that may be mentioned include crosslinked or non-crosslinked polymers of sodium acrylamido-2-methylpropanesulfonate, such as the polymer used in the commercial product Simulgel^{™} 800 (CTFA name: Sodium Polyacryloyldimethyltaurate).

Examples of water-soluble or water-dispersible AMPS^{®} copolymers in accordance with the invention that may be mentioned include:
- acrylamide/sodium acrylamido-2-methylpropanesulfonate crosslinked copolymers, such as the copolymer used in the commercial product Sepigel 305 (CTFA name: Polyacrylamide/C₁₃-C₁₄ Isoparaffin/Laureth-7) or the copolymer used in the commercial product sold under the trade name Simulgel^{™} 600 (CTFA name: Acrylamide/Sodium Acryloyldimethyltaurate/Isohexadecane/Polysorbate-80) by the company SEPPIC;
- copolymers of AMPS^{®} and of vinylpyrrolidone or of vinylformamide, such as the copolymer used in the commercial product sold under the name Aristoflex AVC by the company Clariant (CTFA name: Ammonium Acryloyldimethyltaurate/VP Copolymer) but neutralized with sodium hydroxide or potassium hydroxide;
- copolymers of AMPS^{®} and of sodium acrylate, for instance AMPS^{®}/sodium acrylate copolymer such as the copolymer used in the commercial product sold under the name Simulgel^{™} EG by the company SEPPIC (CTFA name: Acrylamide/Sodium Acryloyldimethyltaurate/Isohexadecane/Polysorbate-80);
- copolymers of AMPS^{®} and of hydroxyethyl acrylate, for instance AMPS^{®}/hydroxyethyl acrylate copolymer, such as the copolymer used in the commercial product sold under the name Simulgel^{™} NS by the company SEPPIC (CTFA name: Hydroxyethyl acrylate/Sodium Acryloyldimethyltaurate copolymer (and) Squalane (and) Polysorbate-60).

The preferred polymers are more particularly sodium acrylamido-2-methylpropanesulfonate homopolymers, such as the homopolymer used in the commercial product Sepigel^{™} 800, and AMPS^{®}/hydroxyethyl acrylate copolymers, such as the copolymer used in the commercial product sold under the name Simulgel NS.

According to one particularly preferred form of the invention, the AMPS^{®} polymer or copolymer will be used in powder form.

The polymers derived from AMPS^{®} may further be those comprising:
- from 80 mol% to 99 mol% of 2-acrylamido-2-methylpropanesulfonic acid (AMPS^{®}) units of formula (3) below: 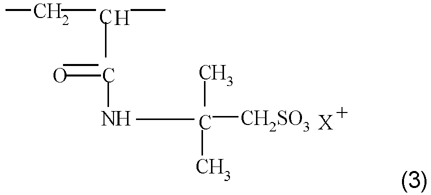 in which X⁺ is a proton, an alkali metal cation, an alkaline-earth metal cation or an ammonium ion; and
- 1 mol% to 20 mol% and preferably from 1 mol% to 15 mol% of units of formula (4) below: 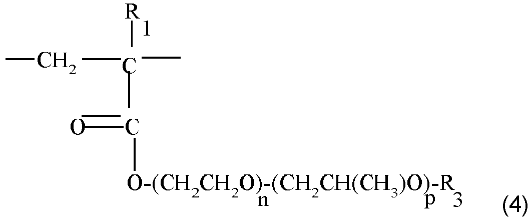 in which n and p, independently of each other, denote a number of moles and ranges from 0 to 30 and preferably from 1 to 20, with the proviso that n + p is less than or equal to 30, preferably less than 25 and better still less than 20; R₁ denotes a hydrogen atom or a linear or branched C₁-C₆ alkyl radical (preferably methyl) and R₃ denotes a linear or branched alkyl comprising m carbon atoms, with m ranging from 6 to 30 and preferably from 10 to 25.

These polymers according to the invention may be obtained according to the standard free-radical polymerization processes in the presence of one or more initiators such as, for example, azobisisobutyronitrile (AIBN), azobisdimethylvaleronitrile, 2,2-azobis[2-amidinopropane] hydrochloride (ABAH = 2,2-azoBis-[2-Amidinopropane] hydrochloride), organic peroxides such as dilauryl peroxide, benzoyl peroxide, tert-butyl hydroperoxide, etc., mineral peroxide compounds such as potassium persulfate or ammonium persulfate, or H₂O₂ optionally in the presence of reducing agents.

The polymers are obtained especially by free-radical polymerization in tert-butanol medium from which they precipitate. Using polymerization in tert-butanol, it is possible to obtain a size distribution of the polymer particles that is particularly favourable for its uses.

The polymerization reaction may be performed at a temperature of between 0 and 150°C, preferably between 20 and 100°C, either at atmospheric pressure or under reduced pressure. It may also be performed under inert atmosphere, and preferably under nitrogen.

It is thus possible to use the polymers prepared from 2-acrylamido-2-methylpropanesulfonic acid (AMPS^{®}), or a sodium or ammonium salt thereof, with an ester of (meth)acrylic acid and:
- of a C₁₀-C₁₈ alcohol oxyethylenated with 8 mol of ethylene oxide (Genapol C-080^{®} from the company Clariant),
- of a C₁₁ oxo alcohol oxyethylenated with 8 mol of ethylene oxide (Genapol UD-080^{®} from the company Clariant),
- of a C₁₁ oxo alcohol oxyethylenated with 7 mol of ethylene oxide (Genapol UD-070^{®} from the company Clariant),
- of a C₁₂-C₁₄ alcohol oxyethylenated with 7 mol of ethylene oxide (Genapol LA-070^{®} from the company Clariant),
- of a C₁₂-C₁₄ alcohol oxyethylenated with 9 mol of ethylene oxide (Genapol LA-090^{®} from the company Clariant),
- of a C₁₂-C₁₄ alcohol oxyethylenated with 11 mol of ethylene oxide (Genapol LA-110^{®} from the company Clariant),
- of a C₁₆-C₁₈ alcohol oxyethylenated with 8 mol of ethylene oxide (Genapol T-080^{®} from the company Clariant),
   of a C₁₆-C₁₈ alcohol oxyethylenated with 11 mol of ethylene oxide (Genapol T-110^{®} from the company Clariant),
- of a C₁₆-C₁₈ alcohol oxyethylenated with 15 mol of ethylene oxide (Genapol T-150^{®} from the company Clariant),
- of a C₁₆-C₁₈ alcohol oxyethylenated with 20 mol of ethylene oxide (Genapol T-200^{®} from the company Clariant),
- of a C₁₆-C₁₈ alcohol oxyethylenated with 25 mol of ethylene oxide (Genapol T-250^{®} from the company Clariant),
- of a C₁₈-C₂₂ alcohol oxyethylenated with 25 mol of ethylene oxide,
- of a C₁₆-C₁₈ iso-alcohol oxyethylenated with 25 mol of ethylene oxide.

According to one preferred embodiment, the polymer is a copolymer of AMPS^{®} and of a C₁₆-C₁₈ alcohol methacrylate comprising from 6 to 25 mol of oxyethylene groups, obtained from methacrylic acid or a methacrylic acid salt and from a C₁₆-C₁₈ alcohol oxyethylenated with 6 to 25 mol of ethylene oxide.

The polymer may also be a copolymer of AMPS^{®} and of a C₁₂-C₁₄ alcohol methacrylate comprising from 6 to 25 mol of oxyethylene groups, obtained from methacrylic acid or a methacrylic acid salt and from a C₁₂-C₁₄ alcohol oxyethylenated with 6 to 25 mol of ethylene oxide.

As polymers of AMPS^{®} type that are preferred according to the present invention, mention may be made of:
- the non-crosslinked copolymer obtained from 92.65 mol% of AMPS^{®} and 7.35 mol% of a C₁₆-C₁₈ alcohol methacrylate comprising 8 oxyethylene groups (Genapol T-080^{®}),
- the non-crosslinked copolymer obtained from 91.5 mol% of AMPS^{®} and 8.5 mol% of a C₁₂-C₁₄ alcohol methacrylate comprising 7 oxyethylene groups (Genapol LA-070^{®}),
- the crosslinked copolymer obtained from 96.45 mol% of AMPS^{®} and 3.55 mol% of a C₁₆-C₁₈ alcohol methacrylate comprising 25 oxyethylene groups (Genapol T-250^{®}), the crosslinking agent is trimethylolpropane triacrylate,
- the crosslinked copolymer obtained from AMPS^{®} and from a C₂₂ alcohol methacrylate comprising 25 oxyethylene groups; this polymer is sold under the name Aristoflex HMB^{®} by the company Clariant,
- the acrylamide/sodium 2-methyl-2-[(1-oxo-2-propenyl)amino] -1-propanesulfonate (AMPS^{®}) copolymer such as Simulgel 600^{®} in the form of an emulsion containing polysorbate 80 as surfactant and containing isohexadecane as fatty phase, sold by the company SEPPIC, or alternatively Simulgel EG^{®}, Simulgel A^{®} and Simulgel 501^{®} sold by the same company.

Simulgel 600^{®} is described especially in document FR 2 785 801. It is more specifically a reverse latex. The AMPS^{®} polyelectrolyte is 2-methyl-2-[(1-oxo-2-propenyl)amino]-1-propanesulfonic acid partially or totally salified especially in sodium salt or ammonium salt form, to a proportion of from 30 mol% to 50 mol% in the mixture comprising the AMPS^{®} and an acrylamide, which is itself in a proportion of 50% to 70%.

For the purpose of the present invention, the preferred polymers derived from AMPS^{®} is the optionally crosslinked and/or neutralized 2-acrylamido-2-methylpropanesulfonic acid homopolymers, for instance the poly(2-acrylamido-2-methylpropanesulfonic acid) sold by the company Clariant under the name Hostacerin AMPS^{®} (CTFA name: ammonium polyacryldimethyltauramide).

### Optionally modified carboxyvinyl polymers

According to an embodiment, the hydrophilic gelling agent is chosen from the optionally modified carboxyvinyl polymers, such as the products marketed under the trade names Carbopol (CTFA name: carbomer) and Pemulen (CTFA name: Acrylates/C 10-30 akyl acrylate crosspolymer) by Goodrich.

According to an embodiment, the modified or unmodified carboxyvinyl polymers may be copolymers derived from the polymerization of at least one monomer (a) chosen from α,β-ethylenically unsaturated carboxylic acids or esters thereof, with at least one ethylenically unsaturated monomer (b) comprising a hydrophobic group.

The term "copolymers" means both copolymers obtained from two types of monomer and those obtained from more than two types of monomer, such as terpolymers obtained from three types of monomer. Their chemical structure more particularly comprises at least one hydrophilic unit and at least one hydrophobic unit. The term "hydrophobic group or unit" means a radical with a saturated or unsaturated, linear or branched hydrocarbon-based chain, comprising at least 8 carbon atoms, preferably from 10 to 30 carbon atoms, in particular from 12 to 30 carbon atoms and more preferentially from 18 to 30 carbon atoms.

Preferably, these copolymers are chosen from copolymers derived from the polymerization:
- of at least one monomer of formula (I) below: 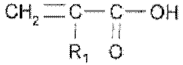 in which R₁ denotes H or CH₃ or C₂H₅, i.e. acrylic acid, methacrylic acid or ethacrylic acid monomers, and
- of at least one monomer of unsaturated carboxylic acid (C₁₀-C₃₀)alkyl ester type corresponding to the monomer of formula (II) below: 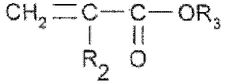 in which R₂ denotes H or CH₃ or C₂H₅ (i.e. acrylate, methacrylate or ethacrylate units) and preferably H (acrylate units) or CH₃ (methacrylate units), R₃ denoting a C₁₀-C₃₀ and preferably C₁₂-C₂₂ alkyl radical.

The unsaturated carboxylic acid (C₁₀-C₃₀)alkyl esters are preferably chosen from lauryl acrylate, stearyl acrylate, decyl acrylate, isodecyl acrylate and dodecyl acrylate, and the corresponding methacrylates, such as lauryl methacrylate, stearyl methacrylate, decyl methacrylate, isodecyl methacrylate and dodecyl methacrylate, and mixtures thereof.

According to a preferred embodiment, these polymers are crosslinked.

Among the copolymers of this type that will be used more particularly are polymers derived from the polymerization of a monomer mixture comprising:
- essentially acrylic acid,
- an ester of formula (II) described above in which R₂ denotes H or CH₃, R₃ denoting an alkyl radical containing from 12 to 22 carbon atoms, and
- a crosslinking agent, which is a well-known copolymerizable unsaturated polyethylenic monomer, such as diallyl phthalate, allyl (meth)acrylate, divinylbenzene, (poly) ethylene glycol dimethacrylate and methylenebisacrylamide.

Among the copolymers of this type, use will more particularly be made of those consisting of from 95% to 60% by weight of acrylic acid (hydrophilic unit), 4% to 40% by weight of C₁₀-C₃₀ alkyl acrylate (hydrophobic unit) and 0 to 6% by weight of crosslinking polymerizable monomer, or alternatively those consisting of from 98% to 96% by weight of acrylic acid (hydrophilic unit), 1% to 4% by weight of C₁₀-C₃₀ alkyl acrylate (hydrophobic unit) and 0.1% to 0.6% by weight of crosslinking polymerizable monomer such as those described previously.

Among the abovementioned polymers, the ones that are most particularly preferred according to the present invention are acrylate/C₁₀-C₃₀-alkyl acrylate copolymers (INCI name: Acrylates/ C₁₀-C₃₀ Alkyl acrylate Crosspolymer) such as the products sold by the company Lubrizol under the trade names Pemulen TR-1, Pemulen TR-2, Carbopol 1382, Carbopol EDT 2020 and Carbopol Ultrez 20 Polymer, and even more preferentially Pemulen TR-2.

Among the modified or unmodified carboxyvinyl polymers, mention may also be made of sodium polyacrylates such as those sold under the name Cosmedia SP^{®} containing 90% solids and 10% water, or Cosmedia SPL^{®} as an inverse emulsion containing about 60% solids, an oil (hydrogenated polydecene) and a surfactant (PPG-5 Laureth-5), both sold by the company Cognis.

Mention may also be made of partially neutralized sodium polyacrylates that are in the form of an inverse emulsion comprising at least one polar oil, for example the product sold under the name Luvigel^{®} EM sold by the company BASF.

The modified or unmodified carboxy vinyl polymers may also be chosen from crosslinked (meth)acrylic acid homopolymers.

For the purposes of the present patent application, the term "(meth) acrylic" means "acrylic or methacrylic".

Examples that may be mentioned include the products sold by Lubrizol under the names Carbopol 910, 934, 940, 941, 934 P, 980, 981, 2984, 5984 and Carbopol Ultrez 10 Polymer, or by 3V-Sigma under the name Synthalen^{®} K, Synthalen^{®} L or Synthalen^{®} M. Among the modified or unmodified carboxyvinyl polymers, mention may be made in particular of Carbopol (INCI name: carbomer) and Pemulen (CTFA name: Acrylates/C₁₀-C₃₀ alkyl acrylate crosspolymer) sold by the company Lubrizol.

According to a preferred embodiment, the amount of the hydrophilic gelling agent may range, for example, from 0.01% to 5% by weight, preferably from 0.1% to 1% by weight, relative to the total weight of the composition.

According to a preferred embodiment, the composition of the present invention, especially the aqueous phase, does not comprise any additional surfactant.

### Adjuvants

In a known manner, the composition of the invention may also contain adjuvants that are common in cosmetics and/or dermatology, such as preserving agents, antioxidants, complexing agents, pH modifiers (acidic or basic), fragrances, fillers, bactericides, odour absorbers, colorants (pigments and dyes), film-forming polymers, additional oils such as silicone oil, or fluoro oils, and active ingredients.

Needless to say, a person skilled in the art will take care to select this or these optional additional compound(s), and/or the amount thereof, such that the properties of the composition according to the invention are not, or are not substantially, adversely affected by the envisaged addition.

### Galenical forms

The compositions according to the invention may be in the form of a lotion, cream, serum pomade or a gel whose viscosity may vary as a function of the desired application, the region of human keratin material to be treated and the desired conditioning.

### Methods and use

According to an embodiment, the present invention relates to a non-therapeutic method for treating a keratin material, comprising the step of applying the composition of the present invention to the keratin material.

Preferably, the present invention relates to a method for caring for the skin, comprising the step of applying the composition of the present invention to the skin.

The present invention also relates to a cosmetic process for treating and/or caring for keratin materials, characterized in that it comprises applying to the surface of the keratin material at least one composition as defined previously.

In the patent application, unless specifically mentioned otherwise, the contents are expressed on a weight basis relative to the total weight of the composition.

The examples that follow are aimed at illustrating the compositions and processes according to this invention, but are not in any way a limitation of the scope of the invention.

All the parts and percentages in the examples are given on a weight basis and all the measurements were obtained at about 25°C, unless otherwise mentioned.

### EXAMPLES

The following compositions are prepared according to the following protocol:
1. Prepare the aqueous phase as follows:
   a. Introduce water in the kettle;
   b. Introduce aqueous polymer with stirring, adjust pH if needed;
   c. Introduce polyol or actives/preservative etc; and
   d. Heat to 80°C;
2. Prepare the fatty phase as follows :
   a. Introduce Hydrophilic gelling agents ;
   b. Introduce all oil/wax/fatty etc. phase; and
   c. Heat to 80°C;
3. Add phase 2 into phase 1 with stirring;
4. Decrease the temp to 50°C;
5. Introduce Fragrance if needed; and
6. Decrease the temp to 30°C.

The following compositions are prepared:

| **INCI name** | **Commercial name (Supplier)** | **Ex.1 (invention)** | **Ex.2 (invention)** | **Ex.3 (comparative)** |
|---|---|---|---|---|
| CAFFEINE | CAFFEINE POWDER (BASF) | 1.6 | - | - |
| MENTHOL | MENTHOL LAEVO PELLETS (SYMRISE) | 0.1 | - | - |
| BLUE 1 | UNICERT BLUE 05601-J (SENSIENT) | 0.0001 | - | - |
| CAPRYLIC/CAPRIC TRIGLYCERIDE | WILFARESTER MCT 7030 (WILMAR) | 5 | 10 | 10 |
| BUTYROSPERMUM PARKII (SHEA) BUTTER | REFINED TRACED SHEA OLEIN (OLVEA) | 2 | - | - |
| SQUALANE | NEOSSANCE SQUALANE (AMYRIS) | 2 | - | - |
| SILICA SILYLATE | DOW CORNING VM-2270 AEROGEL FINE PARTICLES (DOW CORNING) | 0.1 | - | - |

| | | | | |
|---|---|---|---|---|
| FRAGRANCE | WOODY FRESH 22 LO 21240 (FIRMENICH) | 0.15 | - | - |
| HYDROGENATED POLYISOBUTENE | PARLEAM (NOF CORPORATION) | 0.5 | - | - |
| AMMONIUM POLYACRYLOYLDIMETHYL TAURATE | HOSTACERIN AMPS^{®} (CLARIANT) | 0.35 | 0.35 | 0.35 |
| POLYAMIDE-8 | OLEOCRAFT LP-20-PA-(MV) (CRODA) | 2 | 2 | - |
| PHENOXYETHANOL | SEPICIDE LD (SEPPIC) | 0.3 | 0.3 | 0.3 |
| WATER | | QS | QS | QS |
| GLYCERIN | PALMERA G995E (KLK OLEO) | 3 | 3 | 3 |
| CAPRYLYL GLYCOL | HYDROLITE CG (SYMRISE) | 0.2 | 0.2 | 0.2 |

The hydration properties and the stability of the compositions are evaluated according to the following tests:

### Hydration evaluation

The hydration is evaluated by a score comprised being 1 and 5:

| Hydration score | | | | |
|---|---|---|---|---|
| 1 | 2 | 3 | 4 | 5 |
| Extreme Oily | Oily | Normal | Hydration | Extreme Hydration |
| Blind test by expert N=4 Score >= 3 means hydration | | | | |

### Stability test

Pass means:
1. Thick texture but breaks quickly upon application and rubbing with visible spherical water droplets.
2. Stock T24h in room temperature (RT) observe aspect with smooth aspect without oil/water leakage.
3. Stock 1 week in RT, 4°C, 45°C and come back to RT observe aspect with smooth aspect without oil/water leakage

The following results are obtained for examples 1 and 2 (according to the invention) and example 3:

| | Ex.1 (invention) | Ex.2 (invention) | Ex.3 (comparative) |
|---|---|---|---|
| Stability | Pass | Pass | Failed |
| Hydration | 5 | 3 | N/A |

These results show that polyamide-8 is an essential ingredient in order to obtain a stable composition with satisfying hydration properties. Indeed, without polyamide-8, the composition cannot transfer to water droplets on the skin.

### COMPOSITIONS COMPRISING FURTHER HYDROPHOBIC INGREDIENTS

Other compositions (comparative and according to the invention) are prepared:

| **INCI name** | **Commercial name (Supplier)** | **Ex.2 (invention)** | **Ex.4 (comparative)** | **Ex.5 (invention)** | **Ex.6 (invention)** |
|---|---|---|---|---|---|
| CAPRYLIC/CAPRIC TRIGLYCERIDE | WILFARESTER MCT 7030 (WILMAR) | 10 | 0 | 5 | 1 |
| SQUALANE | NEOSSANCE SQUALANE (AMYRIS) | - | 10 | 5 | 9 |
| POLYAMIDE-8 | OLEOCRAFT LP-20-PA-(MV) (CRODA) | 2 | 2 | 2 | 2 |
| AMMONIUM POLYACRYLOYLDIMETHYL TAURATE | HOSTACERIN AMPS^{®} (CLARIANT) | 0.35 | 0.35 | 0.35 | 0.35 |
| PHENOXYETHANOL | SEPICIDE LD (SEPPIC) | 0.3 | 0.3 | 0.3 | 0.3 |
| WATER | | QS | QS | QS | QS |
| GLYCERIN | PALMERA G995E (KLK OLEO) | 3 | 3 | 3 | 3 |
| CAPRYLYL GLYCOL | HYDROLITE CG (SYMRISE) | 0.2 | 0.2 | 0.2 | 0.2 |

The stability and hydration results are as follows:

| | Ex.2 (invention) | Ex.4 (comparative) | Ex.5 (invention) | Ex.6 (invention) |
|---|---|---|---|---|
| Stability | Pass | Failed | Pass | Pass |
| Hydration | 3 | N/A | 4.5 | 4 |

These results show that the absence of triglycerides gives a composition which has not satisfying stability properties.

Examples 5 and 6 show that the addition of squalane in addition to the triglycerides improves the hydration properties of the corresponding compositions.

All these results show that the triglycerides improve the compatibility of polyamide-8 with the lipophilic phase.

Other compositions (comparative and according to the invention) are prepared:

| **INCI name** | **Commercial name (Supplier)** | **Ex.5 (invention)** | **Ex.7 (invention)** | **Ex.8 (comparative)** |
|---|---|---|---|---|
| CAPRYLIC/CAPRIC TRIGLYCERIDE | WILFARESTER MCT 7030 (WILMAR) | 5 | 5 | - |
| SQUALANE | NEOSSANCE SQUALANE (AMYRIS) | 5 | - | - |
| HYDROGENATED POLYISOBUTENE | PARLEAM (NOF CORPORATION) | - | 5 | 10 |
| POLYAMIDE-8 | OLEOCRAFT LP-20-PA-(MV) (CRODA) | 2 | 2 | 2 |
| AMMONIUM POLYACRYLOYLDIMETHYL TAURATE | HOSTACERIN AMPS^{®} (CLARIANT) | 0.35 | 0.35 | 0.35 |
| PHENOXYETHANOL | SEPICIDE LD (SEPPIC) | 0.3 | 0.3 | 0.3 |
| WATER | | QS | QS | QS |
| GLYCERIN | PALMERA G995E (KLK OLEO) | 3 | 3 | 3 |
| CAPRYLYL GLYCOL | HYDROLITE CG (SYMRISE) | 0.2 | 0.2 | 0.2 |

The stability and hydration results are as follows:

| | Ex.5 (invention) | Ex.7 (invention) | Ex.8 (comparative) |
|---|---|---|---|
| Stability | Pass | Pass | Fail |
| Hydration | 4.5 | 3.5 | N/A |

These results show that the presence of hydrogenated polyisobutene is not sufficient to give a composition which has not satisfying stability properties, in the absence of triglycerides (comparative example 8).

These results also show that the presence of C8-C36 saturated or unsaturated carbon chains improves the hydration properties of the corresponding compositions, especially when comparing the hydration score of example 2 (hydration score of 3 without squalane or hydrogenated polyisobutene) with the hydration scores of examples 5 (with squalane) and 7 (with hydrogenated polyisobutene), respectively of 4.5 and 3.5.

Other compositions according to the invention are prepared:

| **INCI name** | **Commercial name (Supplier)** | **Ex.2 (invention)** | **Ex.9 (invention)** | **Ex.10 (invention)** |
|---|---|---|---|---|
| CAPRYLIC/CAPRIC TRIGLYCERIDE | WILFARESTER MCT 7030 (WILMAR) | 10 | 9 | 5 |
| BUTYROSPERMUM PARKII (SHEA) BUTTER | REFINED TRACED SHEA OLEIN (OLVEA) | - | - | 5 |
| SYNTHETIC WAX | CIREBELLE 303 (CIREBELLE) | - | 1 | - |
| POLYAMIDE-8 | OLEOCRAFT LP-20-PA-(MV) (CRODA) | 2 | 2 | 2 |
| AMMONIUM POLYACRYLOYLDIMETHYL TAURATE | HOSTACERIN AMPS^{®} (CLARIANT) | 0.35 | 0.35 | 0.35 |
| PHENOXYETHANOL | SEPICIDE LD (SEPPIC) | 0.3 | 0.3 | 0.3 |
| WATER | | QS | QS | QS |
| GLYCERIN | PALMERA G995E (KLK OLEO) | 3 | 3 | 3 |
| CAPRYLYL GLYCOL | HYDROLITE CG (SYMRISE) | 0.2 | 0.2 | 0.2 |

The stability and hydration results are as follows:

| | Ex.2 (invention) | Ex.9 (invention) | Ex.10 (invention) |
|---|---|---|---|
| Stability | Pass | Pass | Pass |
| Hydration | 3 | 3.5 | 4 |

These results show that the addition of other lipophilic ingredients such as shea butter or synthetic wax is also compatible with glycerides and gives satisfying stability and hydration properties.

### USE OF OTHER TRIGLYCERIDES

Other compositions (comparative and according to the invention) are prepared:

| **INCI name** | **Commercial name (Supplier)** | **Ex.2 (invention)** | **Ex.11 (invention)** | **Ex.12 (invention)** |
|---|---|---|---|---|
| CAPRYLIC/CAPRIC TRIGLYCERIDE | WILFARESTER MCT 7030 (WILMAR) | 10 | - | - |
| C18-36 ACID TRIGLYCERIDE | DUB TGI 24 (STEARINERIE DUBOIS) | - | 10 | 5 |
| SQUALANE | NEOSSANCE SQUALANE (AMYRIS) | - | - | 5 |
| POLYAMIDE-8 | OLEOCRAFT LP-20-PA-(MV) (CRODA) | 2 | 2 | 2 |
| AMMONIUM POLYACRYLOYLDIMETHYL TAURATE | HOSTACERIN AMPS^{®} (CLARIANT) | 0.35 | 0.35 | 0.35 |
| PHENOXYETHANOL | SEPICIDE LD (SEPPIC) | 0.3 | 0.3 | 0.3 |
| WATER | | QS | QS | QS |
| GLYCERIN | PALMERA G995E (KLK OLEO) | 3 | 3 | 3 |
| CAPRYLYL GLYCOL | HYDROLITE CG (SYMRISE) | 0.2 | 0.2 | 0.2 |

The stability and hydration results are as follows:

| | Ex.2 (invention) | Ex.11 (invention) | Ex.12 (invention) |
|---|---|---|---|
| Stability | Pass | Pass | Pass |
| Hydration | 3 | 3 | 4 |

These results show that several kinds of triglycerides improve the compatibility of polyamide-8 with the lipophilic phase.

Example 12 shows that the addition of squalane in addition to the triglycerides improves the hydration properties of the corresponding compositions.

### INFLUENCE OF THE NATURE OF THE HYDROPHILIC GELLING AGENT

Other compositions (comparative and according to the invention) are prepared:

| **INCI name** | **Commercial name (Supplier)** | **Ex.13 (invention)** | **Ex.14 (invention)** | **Ex.15 (invention)** |
|---|---|---|---|---|
| CAPRYLIC/CAPRIC TRIGLYCERIDE | WILFARESTER MCT 7030 (WILMAR) | 5 | 5 | 5 |
| BUTYROSPERMUM PARKII (SHEA) BUTTER | REFINED TRACED SHEA OLEIN (OLVEA) | 2 | 2 | 2 |
| SQUALANE | NEOSSANCE SQUALANE (AMYRIS) | 2 | 2 | 2 |
| HYDROGENATED POLYISOBUTENE | PARLEAM (NOF CORPORATION) | 0.5 | 0.5 | 0.5 |
| POLY AM IDE-8 | OLEOCRAFT LP-20-PA-(MV) (CRODA) | 2 | 2 | 2 |
| AMMONIUM POLYACRYLOYLDIMETHYL TAURATE | HOSTACERIN AMPS^{®} (CLARIANT) | 0.35 | - | - |
| CARBOMER | CARBOPOL 980 (LUBRIZOL) | - | 0.35 | - |
| XANTHAN GUM | RHODICARE XC RHODIA (SOLVAY) | - | - | 0.35 |
| PHENOXYETHANOL | SEPICIDE LD (SEPPIC) | 0.3 | 0.3 | 0.3 |
| WATER | | QS | QS | QS |
| GLYCERIN | PALMERA G995E (KLK OLEO) | 3 | 3 | 3 |
| CAPRYLYL GLYCOL | HYDROLITE CG (SYMRISE) | 0.2 | 0.2 | 0.2 |

The stability and hydration results are as follows:

| | Ex.13 (invention) | Ex.14 (invention) | Ex.15 (comparative) |
|---|---|---|---|
| Stability | Pass | Pass | Fail |
| Hydration | 5 | 4.5 | N/A |

These results show that the use of carbomer or AMPS^{®} gives a composition with satisfying stability and hydration properties.

They also show that the use of xanthan gum (hydrophilic gelling agent out of the invention) does not give a stable composition (comparative example 15).

## Claims

1. A composition, preferably a cosmetic composition, in the form of an oil-in-water emulsion, comprising:
- a dispersed fatty phase comprising:
. at least one triglyceride made of glycerol and fatty acids, preferably at least one triglyceride made of glycerol and fatty acids with saturated or unsaturated, linear or branched, chains comprising from 8 to 36 carbon atoms, and
. at least one hydrophobic polymer selected from the ester-terminated poly(ester-amide)s;
wherein said dispersed fatty phase comprises at least 10% by weight of triglyceride(s) in relation to the total weight of said dispersed fatty phase; and
- a continuous aqueous phase comprising at least one hydrophilic gelling agent selected from the group consisting of the polymers comprising at least one monomer being acrylamido-2-methylpropanesulfonic acid, the optionally modified carboxyvinyl polymers, and mixtures thereof.

2. The composition of claim 1, wherein the amount of triglyceride(s) is comprised from 10% to 90%, preferably from 30% to 70%, and more preferably from 40% to 60%, by weight of triglyceride(s) in relation to the total weight of said dispersed fatty phase.

3. The composition of claim 1 or 2, wherein the dispersed fatty phase further comprises at least one hydrophobic additional ingredient.

4. The composition of claim 3, wherein the hydrophobic additional ingredient is chosen from the linear or branched hydrocarbons of mineral or synthetic origin such as squalane, the plant oils such as shea butter, and mixtures thereof.

5. The composition of any of claims 1 to 4, wherein the hydrophobic polymer is an ester-terminated poly(ester-amide) comprising at least one dimer dilinoleate unit, said hydrophobic polymer being preferably a bis-stearyl ethylenediamine/neopentyl glycol/hydrogenated dimer dilinoleate copolymer.

6. The composition of any of claims 1 to 5, wherein the amount of the hydrophobic polymer is comprised from 0.3% to 10% by weight, more preferably from 0.5% to 5% by weight, and most preferably from 1% to 3% by weight, based on the total weight of the composition.

7. The composition of any of claims 1 to 6, wherein the hydrophilic gelling agent is chosen from polymers of which at least one monomer of 2-acrylamido-2-methylpropanesulfonic acid is selected from the group consisting of crosslinked or non-crosslinked homopolymers or copolymers comprising at least the acrylamido-2-methylpropanesulfonic acid monomer, in a form partially or totally neutralized with a mineral base other than ammonia, such as sodium hydroxide or potassium hydroxide.

8. The composition of claim 7, wherein the hydrophilic gelling agent is selected from optionally crosslinked and/or neutralized 2-acrylamido-2-methylpropanesulfonic acid homopolymers, said hydrophilic gelling agent being preferably the poly(2-acrylamido-2-methylpropanesulfonic acid).

9. The composition of any of claims 1 to 8, wherein the amount of the hydrophilic gelling agent is comprised from 0.01% to 5% by weight, preferably from 0.1% to 1% by weight, relative to the total weight of the composition.

10. The composition of any of claims 1 to 9, wherein the aqueous phase further comprises at least one water-miscible organic solvent.

11. The composition of any of claims 1 to 10, wherein the amount of dispersed fatty phase is less than or equal to 30% by weight relative to the total weight of the composition.

12. The composition of any of claims 1 to 11, wherein the amount of continuous aqueous phase is greater than or equal to 50% by weight relative to the total weight of the composition.

13. A method for caring for the skin, comprising the application to the skin of the composition of any of claims 1 to 12.

## Patentansprüche

1. Zusammensetzung, vorzugsweise eine kosmetische Zusammensetzung, in Form einer Öl-in-Wasser-Emulsion, umfassend:
- eine dispergierte Fettphase, umfassend:
. mindestens ein Triglycerid aus Glycerin und Fettsäuren, vorzugsweise mindestens ein Triglycerid aus Glycerin und Fettsäuren mit gesättigten oder ungesättigten, linearen oder verzweigten Ketten, umfassend 8 bis 36 Kohlenstoffatome, und
. mindestens ein hydrophobes Polymer, ausgewählt aus den Esterterminierten Poly(ester-amiden);
wobei die dispergierte Fettphase mindestens 10 Gewichts-% Triglycerid(e), bezogen auf das Gesamtgewicht der dispergierten Fettphase, umfasst; und
- eine kontinuierliche wässrige Phase, umfassend mindestens ein hydrophiles Geliermittel, das ausgewählt ist aus der Gruppe, bestehend aus den Polymeren, umfassend mindestens ein Monomer, das Acrylamido-2-methylpropansulfonsäure ist, die optional modifizierten Carboxyvinylpolymere und deren Gemische.

2. Zusammensetzung nach Anspruch 1, wobei die Menge an Triglycerid(en) von 10 bis 90 Gewichts-%, vorzugsweise von 30 bis 70 Gewichts-% und bevorzugter von 40 bis 60 Gewichts-% Triglycerid(e), bezogen auf das Gesamtgewicht der dispergierten Fettphase, ist.

3. Zusammensetzung nach Anspruch 1 oder 2, wobei die dispergierte Fettphase ferner mindestens einen hydrophoben zusätzlichen Bestandteil umfasst.

4. Zusammensetzung nach Anspruch 3, wobei der hydrophobe zusätzliche Bestandteil ausgewählt ist aus linearen oder verzweigten Kohlenwasserstoffen mineralischen oder synthetischen Ursprungs, wie Squalan, Pflanzenölen, wie beispielsweise Sheabutter, und Gemischen davon.

5. Zusammensetzung nach einem der Ansprüche 1 bis 4, wobei das hydrophobe Polymer ein Ester-terminiertes Poly(esteramid) ist, umfassend mindestens eine Dimerdilinoleat-Einheit, wobei das hydrophobe Polymer vorzugsweise ein Bisstearylethylendiamin/Neopentylglykol/hydriertes Dimerdilinoleat-Copolymer ist.

6. Zusammensetzung nach einem der Ansprüche 1 bis 5, wobei die Menge des hydrophoben Polymers von 0,3 bis 10 Gewichts-%, bevorzugter von 0,5 bis 5 Gewichts-% und am meisten bevorzugt von 1 bis 3 Gewichts-%, bezogen auf das Gesamtgewicht der Zusammensetzung, ist.

7. Zusammensetzung nach einem der Ansprüche 1 bis 6, wobei das hydrophile Geliermittel ausgewählt ist aus Polymeren, wovon mindestens ein 2-Acrylamido-2-methylpropansulfonsäure-Monomer ausgewählt ist aus der Gruppe, bestehend aus vernetzten oder nicht vernetzten Homopolymeren oder Copolymeren, umfassend mindestens das Acrylamido-2-methylpropansulfonsäure-Monomer in einer Form, die teilweise oder vollständig mit einer mineralischen Base außer Ammoniak, wie Natriumhydroxid oder Kaliumhydroxid, neutralisiert ist.

8. Zusammensetzung nach Anspruch 7, wobei das hydrophile Geliermittel ausgewählt ist aus optional vernetzten und/oder neutralisierten 2-Acrylamido-2-methylpropansulfonsäure-Homopolymeren, wobei das hydrophile Geliermittel vorzugsweise die Poly(2-acrylamido-2-methylpropansulfonsäure) ist.

9. Zusammensetzung nach einem der Ansprüche 1 bis 8, wobei die Menge des hydrophilen Geliermittels von 0,01 bis 5 Gewichts-%, vorzugsweise von 0,1 bis 1 Gewichts-%, bezogen auf das Gesamtgewicht der Zusammensetzung ist.

10. Zusammensetzung nach einem der Ansprüche 1 bis 9, wobei die wässrige Phase ferner mindestens ein mit Wasser mischbares organisches Lösungsmittel umfasst.

11. Zusammensetzung nach einem der Ansprüche 1 bis 10, wobei die Menge an dispergierter Fettphase weniger als oder gleich wie 30 Gewichts-%, bezogen auf das Gesamtgewicht der Zusammensetzung, ist.

12. Zusammensetzung nach einem der Ansprüche 1 bis 11, wobei die Menge an kontinuierlichen wässriger Phase mehr als oder gleich wie 50 Gewichts-%, bezogen auf das Gesamtgewicht der Zusammensetzung, ist.

13. Pflegeverfahren für die Haut, umfassend das Auftragen der Zusammensetzung nach einem der Ansprüche 1 bis 12 auf die Haut.

## Revendications

1. Composition, de préférence composition cosmétique, sous forme d'une émulsion d'huile dans l'eau, comprenant :
- une phase grasse dispersée comprenant :
. au moins un triglycéride composé de glycérol et d'acides gras, de préférence au moins un triglycéride composé de glycérol et d'acides gras à chaînes linéaires ou ramifiées, saturées ou insaturées, comprenant de 8 à 36 atomes de carbone, et
. au moins un polymère hydrophobe choisi parmi les poly(ester-amides) à terminaison ester ;
dans laquelle ladite phase grasse dispersée comprend au moins 10 % en poids de triglycéride(s) par rapport au poids total de ladite phase grasse dispersée ; et
- une phase aqueuse continue comprenant au moins un agent gélifiant hydrophile choisi dans le groupe constitué par les polymères comprenant au moins un monomère étant l'acide acrylamido-2-méthylpropanesulfonique, les polymères carboxyvinyliques facultativement modifiés et les mélanges de ceux-ci.

2. Composition selon la revendication 1, dans laquelle la quantité de triglycéride(s) va de 10% à 90 %, de préférence de 30 % à 70 %, et plus préférentiellement de 40 % à 60 %, en poids de triglycéride(s) par rapport au poids total de ladite phase grasse dispersée.

3. Composition selon la revendication 1 ou 2, dans laquelle la phase grasse dispersée comprend en outre au moins un ingrédient supplémentaire hydrophobe.

4. Composition selon la revendication 3, dans laquelle l'ingrédient hydrophobe supplémentaire est choisi parmi les hydrocarbures linéaires ou ramifiés d'origine minérale ou synthétique tels que le squalane, les huiles végétales telles que le beurre de karité, et les mélanges de ceux-ci.

5. Composition selon l'une quelconque des revendications 1 à 4, dans laquelle le polymère hydrophobe est un poly(ester-amide) à terminaison ester comprenant au moins une unité de dilinoléate dimère, ledit polymère hydrophobe étant de préférence un copolymère de bis-stearyl éthylènediamine/néopentylglycol/dilinoléate dimère hydrogéné.

6. Composition selon l'une quelconque des revendications 1 à 5, dans laquelle la quantité du polymère hydrophobe est de 0,3 % à 10 % en poids, de préférence de 0,5 % à 5 % en poids, et de manière davantage préférée de 1 % à 3 % en poids, sur la base du poids total de la composition.

7. Composition selon l'une quelconque des revendications 1 à 6, dans laquelle l'agent gélifiant hydrophile est choisi parmi les polymères dont au moins un monomère de l'acide 2-acrylamido-2-méthylpropanesulfonique est choisi dans le groupe constitué par les homopolymères ou copolymères réticulés ou non réticulés comprenant au moins le monomère de l'acide acrylamido-2-méthylpropanesulfonique, sous une forme partiellement ou totalement neutralisée par une base minérale autre que l'ammoniaque, telle que l'hydroxyde de sodium ou l'hydroxyde de potassium.

8. Composition selon la revendication 7, dans laquelle l'agent gélifiant hydrophile est choisi parmi les homopolymères de l'acide 2-acrylamido-2-méthylpropanesulfonique facultativement réticulés et/ou neutralisés, ledit agent gélifiant hydrophile étant de préférence le poly(acide 2-acrylamido-2-méthylpropanesulfonique).

9. Composition selon l'une quelconque des revendications 1 à 8, dans laquelle la quantité de l'agent gélifiant hydrophile est de 0,01 % à 5 % en poids, de préférence de 0,1 % à 1 % en poids, par rapport au poids total de la composition.

10. Composition selon l'une quelconque des revendications 1 à 9, dans laquelle la phase aqueuse comprend en outre au moins un solvant organique miscible à l'eau.

11. Composition selon l'une quelconque des revendications 1 à 10, dans laquelle la quantité de phase grasse dispersée est inférieure ou égale à 30 % en poids par rapport au poids total de la composition.

12. Composition selon l'une quelconque des revendications 1 à 11, dans laquelle la quantité de phase aqueuse continue est supérieure ou égale à 50 % en poids par rapport au poids total de la composition.

13. Procédé de soin de la peau comprenant l'application sur la peau de la composition selon l'une quelconque des revendications 1 à 12.
